(19) Europäisches Patentamt
European Patent Office
Office européen des brevets

(11) Publication number: **0 316 712**
**A2**

(12) # EUROPEAN PATENT APPLICATION

(21) Application number: **88118558.1**

(51) Int. Cl.[4]: **C07K 9/00**

(22) Date of filing: **08.11.88**

Claims for the following Contracting States: ES + GR.

(30) Priority: **17.11.87 GB 8726859**

(43) Date of publication of application: **24.05.89 Bulletin 89/21**

(84) Designated Contracting States: **AT BE CH DE ES FR GB GR IT LI LU NL SE**

(71) Applicant: **GRUPPO LEPETIT S.P.A.**
**23, Via G. Murat**
**I-20159 Milano(IT)**

(72) Inventor: **Malabarba, Adriano**
**5/A, Via Roma**
**I-20082 Binasco (MI)(IT)**
Inventor: **Spreafico, Franca**
**9, Via San Francesco d'Assisi**
**I-22050 Pescate (CO)(IT)**
Inventor: **Tarzia, Giorgio**
**3, Vicolo San Clemente**
**I-37100 Verona(IT)**

(74) Representative: **Macchetta, Francesco et al**
**Gruppo Lepetit S.p.A. Patent and Trademark Department 34, Via Roberto Lepetit**
**I-21040 Gerenzano (Varese)(IT)**

(54) **22-Dechloroteicoplanins.**

(57) The present invention is directed to new 22-dechloro derivatives of the antibiotic teicoplanin, to a method for their preparation and to the pharmaceutical compositions containing them.

EP 0 316 712 A2

## 22-DECHLOROTEICOPLANINS

The present invention is directed to new 22-dechloro derivatives of the antibiotic teicoplanin, to a method for their preparation and to the pharmaceutical compositions containing them.

More particularly, the compounds of the invention which possess antimicrobial activity in particular against gram positive bacteria, have the following formula I:

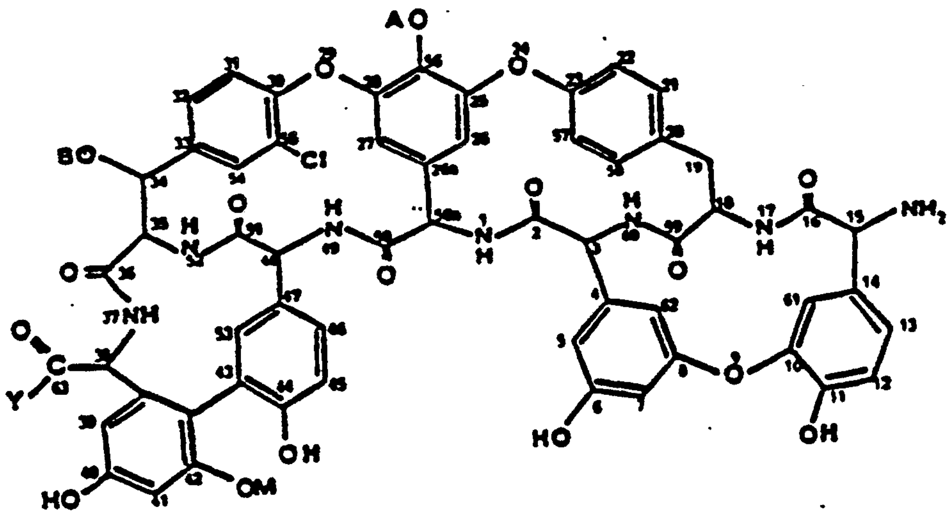

I

wherein:

Y represents hydroxy, an ester function or an amide function,
A represents hydrogen or -N[($C_{10}$-$C_{11}$)aliphatic acyl]-beta-D-2-deoxy-2-amino-glucopyranosyl,
B represents hydrogen or N-acetyl-beta-D-2-deoxy-amino-glucopyranosyl
M represents hydrogen or alpha-D-mannopyranosyl;

and the addition salts thereof.

Teicoplanin is the international non-proprietary name (INN) of the antibiotic substance formerly named teichomycin which is obtained by cultivating the strain Actinoplanes teichomyceticus nov. sp. ATCC 31121 in a culture medium containing assimilable sources of carbon, nitrogen and inorganic salts (see U.S. Patent No. 4,239,751). According to the procedure described in the above cited patent an antibiotic complex containing Teichomycin $A_1$, $A_2$ and $A_3$ is recovered from the separated fermentation broth by extraction with a suitable water insoluble organic solvent and precipitation from the extracting solvent according to common procedures.

Teichomycin $A_2$, which is the major factor of the isolated antibiotic complex, is then separated from the other factors by means of column chromatography on Sephadex ®.

British Patent Application Publication No. 2121401 discloses that antibiotic Teichomycin $A_2$ actually is a mixture of five closely related co-produced main components.

According to recent structural studies it is possible to represent teicoplanin $A_2$ (formerly Teichomycin $A_2$) main components 1, 2, 3, 4 and 5 by the following formula II:

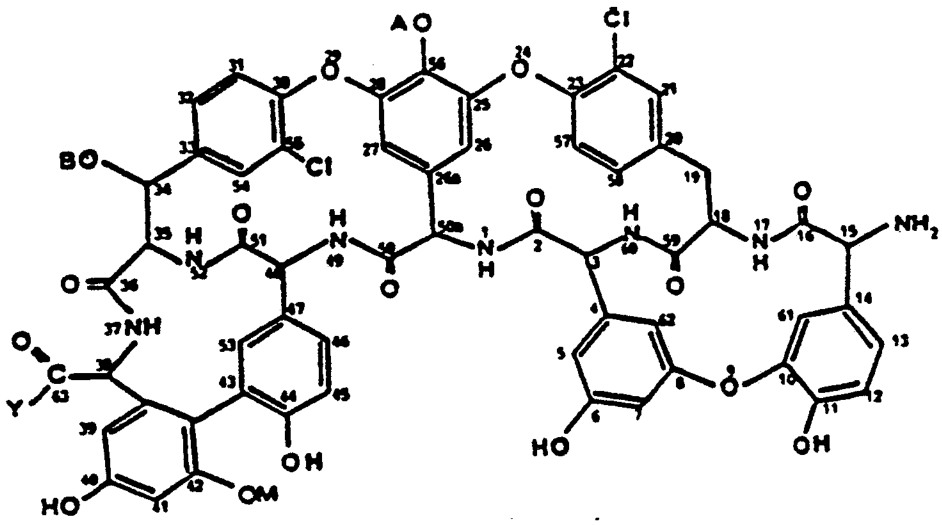

II

wherein

Y is hydroxy,
A represents -N[($C_{10}$-$C_{11}$)aliphatic acyl] -beta-D-2-deoxy-2-amino-glucopyranosyl,
B represent N-acetyl-beta-D-2-deoxy-2-amino-glucopyranosyl,
M represents alpha-D-mannopyranosyl.

More particularly, in teicoplanin $A_2$ component 1, the [($C_{10}$-$C_{11}$)-aliphatic acyl] substituent represents Z-decenoyl, in teicoplanin $A_2$ component 2 represents 8-methyl-nonanoyl, in teicoplanin $A_2$ component 3 represents decanoyl, in teicoplanin $A_2$ component 4 represents 8-methyldecanoyl, in teicoplanin $A_2$ component 5 represents 9-methyldecanoyl.

All the sugar moieties, when present, are linked to the teicoplanin nucleus through O-glycosidic bonds.

In addition, it has been found that it is possible to transform teicoplanin, a pure factor thereof or a mixture of any of said factors in any proportion, into unitary antibiotic products by means of selective hydrolysis of one or two sugar moieties. They are named antibiotic L 17054 and antibiotic L 17046 and are described in European Patent Application Publication No. 119575 and European Patent Application Publication No. 119574, respectively.

Antibiotic L 17054 is represented by the above formula II wherein Y is hydroxy, A represents hydrogen, B represents N-acetyl-beta-D-2-deoxy-2-amino-glucopyranosyl, M represents alpha-D-mannopyranosyl wherein the sugar moieties are linked to the peptidic nucleus through a O-glycosidic bonds.

Antibiotic L 17046 is represented by the above formula II wherein Y is hydroxy, A and M represent hydrogen atoms, and B is N-acetyl-beta-D-2-deoxy-2-amino-glucopyranosyl wherein the sugar moiety is linked to the peptidic nucleus through an O-glycosidic bond.

The compounds of formula II wherein Y represents hydroxy, A represents -N[($C_{10}$-$C_{11}$)aliphatic acyl]-beta-D-2-deoxy-2-amino-glucopyranosyl and B represents N-acetyl-beta-D-2-deoxy-amino-glucopyranosyl are disclosed in European Patent Application No. 88110195.0.

The complete selective cleavage of all the sugar moieties of the teicoplanin compounds gives an aglycone molecule which is called antibiotic L 17392, or deglucoteicoplanin, and is represented by the above formula II wherein Y is hydroxy, and A, B, and M each individually represents a hydrogen group. This selective hydrolysis process is described in European patent application Publication No. 146053.

A substance having the same structural formula is disclosed in European Patent Application Publication No. 90578 and is named antibiotic A 41030 factor B.

This substance is obtained by means of a microbiological process which involves the fermentation of the strain Streptomyces virginiae NRRL 12525 or Streptomyces virginiae NRRL 15156 in a suitable medium, the isolation, purification and separation into its components of antibiotic A 41030, an antibiotic complex of at least seven factors, antibiotic A 41030 factor B, included.

Compounds of formula II wherein Y represents an ester function are described in EP-A- 216775 and EP-A- 182157.

Compounds of formula II wherein Y represents an amide function are described in EP-A-218099.

More particularly, EP-A- 216775 describes compounds of formula II wherein Y represents OR wherein

in turn R represents:
"$(C_1-C_{12})$alkyl, hydroxy$(C_1-C_{12})$alkyl, $(C_1-C_3)$alkoxy$(C_1-C_{12})$alkyl, halo$(C_1-C_{12})$alkyl; a group of formula

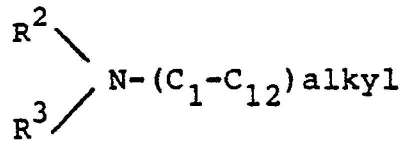

wherein $R^2$ and $R^3$ each independently represents hydrogen or $(C_1-C_4)$alkyl groups, or $R^2$ and $R^3$ taken together with the adjacent nitrogen atom represent a 5-7 membered aromatic, partially hydrogenated or saturated heterocycle ring which may optionally contain a further heteroatom selected from S, O and N; a group of formula

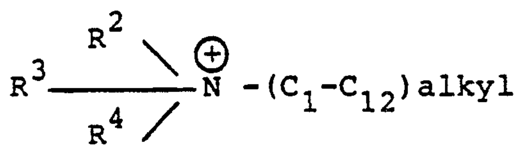

wherein $R^2$ and $R^3$ are as defined above and $R^4$ represents hydrogen or $(C_1-C_4)$alkyl; or R represents a group of formula

$H-[O(CH_2)_m]-_n$
$(C_1-C_3)alkyl[O(CH_2)_m]-_n$
wherein m represents the integer 2 or 3, n is an integer from 1 to 10, and one of the hydrogen atoms of the $-(CH_2)$-group may be substituted by a methyl group; $(C_2-C_{10})$alkanoyloxymethyl, phenyl, substituted phenyl, phenyl$(C_1-C_6)$alkyl, substituted phenyl$(C_1-C_6)$alkyl".
EP-A-218099 gives the following definition for Y:
"Y represents a group

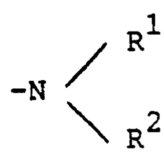

wherein:

$R^1$ represents hydrogen, $(C_1-C_6)$alkyl, hydroxy$(C_2-C_4)$alkyl, halogeno$(C_2-C_4)$alkyl, $(C_1-C_4)$alkoxy$(C_2-C_4)$alkyl, amino$(C_2-C_4)$alkyl, $(C_1-C_4)$alkylamino$(C_2-C_4)$alkyl, di$(C_1-C_4)$alkylamino$(C_2-C_4)$alkyl

$R^2$ represents hydrogen, $(C_1-C_6)$alkyl, hydroxy$(C_2-C_4)$alkyl, halogeno$(C_2-C_4)$alkyl, $(C_1-C_4)$alkoxy$(C_2-C_4)$alkyl, a nitrogen containing 5-6 membered heterocyclic ring

which may be unsaturated, partially saturated or wholly saturated and may contain 1 to 3 further heteroatoms selected from N, S and O wherein 1 to 3 of the ring carbons may optionally bear $(C_1-C_4)$alkyl substituents and one of the ring nitrogens may optionally bear a substituent $R^5$ selected from $(C_1-C_4)$alkyl, $(C_4-C_7)$cycloalkyl, phenyl optionally substituted with halogen or $(C_1-C_4)$alkyl, phenyl$(C_1-C_4)$alkyl, pyridyl, $(C_1-C_4)$alkylpyridinio, and when the ring is wholly saturated two of the ring members may optionally be bridged by an alkylene chain of 1 to 3 carbon atoms wherein one of the methylene groups may optionally be replaced by -NH- or -N $[(C_1-C_4)alkyl]$;
a group -alk-W wherein "alk" represents a linear alkylene chain of 1 to 8 carbon atoms which is optionally substituted with a substituent selected from $(C_1-C_4)$alkyl, hydroxy$(C_1-C_4)$alkyl, hydroxy, carboxy, aminocarbonyl, $(C_1-C_4)$alkylaminocarbonyl, di$(C_1-C_4)$alkylaminocarbonyl, $(C_1-C_4)$alkoxycarbonyl, phenyl$(C_1-C_4)$-

alkoxycarbonyl, and W represents a carboxy, $(C_1-C_4)$alkoxycarbonyl, phenyl$(C_1-C_4)$alkoxycarbonyl, aminocarbonyl, $(C_1-C_4)$aminocarbonyl, di$(C_1-C_4)$aminocarbonyl, pentosaminocarbonyl, hexosaminocarbonyl, ureido, guanidino, a nitrogen containing 5-6 membered heterocyclic ring defined as above, a group of the formula

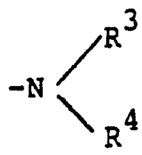

wherein $R^3$ and $R^4$ each independently represent hydrogen, $(C_1-C_6)$alkyl, hydroxy$(C_2-C_4)$alkyl and halogeno$(C_2-C_4)$alkyl, or $R^4$ represents phenylmethyloxycarbonyl and $R^3$ represents hydrogen; a group of the formula

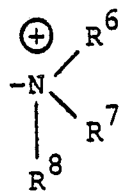

wherein $R^6$ , $R^7$ and $R^8$ each independently represent a $(C_1-C_4)$alkyl,
or $R^1$ and $R^2$ taken together with the adjacent nitrogen atom represent a saturated 5-7 membered heterocyclic ring which may optionally bear one to two $(C_1-C_4)$alkyl substituents on the ring carbons and may contain a further heterogroup selected from -O-, -S-, and $-NR^5-$ wherein $R^5$ is defined as above;

with the proviso that when W represents a group

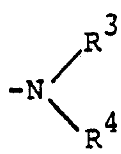

,

a group

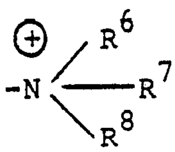

,

ureido, guanidino or a nitrogen containing 5-6 membered heterocyclic ring as defined above directly connected with the "alk" moiety through a bond with a ring nitrogen atom, the linear alkylene "alk" moiety must be of at least two carbon atoms".

EP-A- 152902 describes the selective hydrogenation of teicoplanin $A_2$ component 1 to transform it into teicoplanin $A_2$ component 3 in the presence of a poisoned catalyst such as Palladium on Barium sulfate, Platinum on Barium sulfate, the Lindlar catalyst (Palladium on calcium carbonate poisoned with lead), and 5% (w/w) Palladium sulfide on carbon.

No dechlorination is reported under these conditions.

Harris C.M. et al., in J. Am. Chem. Soc. 107, (1985), pages 6652-6658 describe the preparation of mono- and didechloro derivatives of vancomycin, a known glycopeptidic antibiotic. However, the reaction conditions described therein, mainly hydrogenation in the presence of 10% Pd/C, are not suitable for the

present substrates since they are not selective for the mono-dechloroderivative (i.e. the 22-dechloroteicoplanin derivative) but also lead to the formation of significant amounts of didechloro derivative (i.e. 22,55-didechloroteicoplanin derivative) as will be more fully exemplified below.

One object of the invention is therefore a selective process for removing the chloro-atom in position 22 of the peptidic nucleus of teicoplanin or a teicoplanin derivative.

A further object of this invention is represented by the 22-dechloroteicoplanin derivatives which are obtained by the method of the invention starting from any of the known teicoplanin derivatives.

These compounds maintain the antimicrobial pattern of the starting compounds and are therefore mainly active against gram-positive bacteria.

The present invention encompasses also the pharmaceutical formulations wherein a compound of the invention is employed as the active ingredient, as well as the use of this pharmacologically active substance for treating infections caused by susceptible bacteria.

A preferred group of compounds of the invention is represented by those compounds of formula I wherein Y represents a group

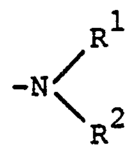

wherein

$R^1$ represents hydrogen, $(C_1\text{-}C_6)$alkyl,

$R^2$ represents $(C_1\text{-}C_6)$alkyl, a nitrogen containing 5-6 membered heterocyclic ring which may be unsaturated, partially saturated or wholly saturated and may contain 1 to 3 further heteroatoms selected from N, S and O wherein 1 to 3 of the ring carbons may optionally bear $(C_1\text{-}C_4)$alkyl substituents and one of the ring nitrogens may optionally bear a substituent $R^5$ selected from $(C_1\text{-}C_4)$alkyl, $(C_4\text{-}C_7)$cycloalkyl, phenyl, and pyridyl;

a wholly saturated nitrogen containing 5-6 membered heterocyclic ring which may contain a further N atom wherein 1 to 3 of the ring carbons may optionally bear $(C_1\text{-}C_4)$alkyl substituents, one of the ring nitrogens may optionally bear a substituent $R^5$ representing $(C_1\text{-}C_4)$alkyl and two of the ring members are bridged by an alkylene chain of 1 to 3 carbon atoms wherein one of the methylene groups may optionally be replaced by -NH-or - N [$(C_1\text{-}C_4)$alkyl];

a group -alk-W wherein "alk" represents a linear alkylene chain of 1 to 8 carbon atoms which is optionally substituted with a substituent selected from $(C_1\text{-}C_4)$alkyl, carboxy, aminocarbonyl, $(C_1\text{-}C_4)$-alkylaminocarbonyl, di$(C_1\text{-}C_4)$alkylaminocarbonyl, $(C_1\text{-}C_4)$alkoxycarbonyl, phenyl$(C_1\text{-}C_4)$alkoxycarbonyl, and W represents a carboxy, $(C_1\text{-}C_4)$alkoxycarbonyl, phenyl$(C_1\text{-}C_4)$alkoxycarbonyl, aminocarbonyl, $(C_1\text{-}C_4)$-aminocarbonyl, di$(C_1\text{-}C_4)$aminocarbonyl, glucosaminocarbonyl, ureido, guanidino, a nitrogen containing 5-6 membered heterocyclic ring which may be unsaturated, partially saturated or wholly saturated and may contain 1 to 3 further heteroatoms selected from N, S and O wherein 1 to 3 of the ring carbons may optionally bear $(C_1\text{-}C_4)$alkyl substituents and one of the ring nitrogens may optionally bear a substituent $R^5$ selected from $(C_1\text{-}C_4)$alkyl, $(C_4\text{-}C_7)$cycloalkyl, phenyl, and pyridyl; a wholly saturated nitrogen containing 5-6 membered heterocyclic ring which may contain a further N atom wherein 1 to 3 of the ring carbons may optionally bear $(C_1\text{-}C_4)$alkyl substituents, one of the ring nitrogens may optionally bear a substituent $R^5$ representing $(C_1\text{-}C_4)$alkyl and two of the ring members are bridged by an alkylene chain of 1 to 3 carbon atoms wherein one of the methylene groups may optionally be replaced by -NH- or - N [$(C_1\text{-}C_4)$alkyl]; a group of the formula

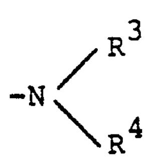

wherein $R^3$ and $R^4$ each independently represent hydrogen, $(C_1\text{-}C_6)$alkyl, hydroxy$(C_2\text{-}C_4)$alkyl and halogeno$(C_2\text{-}C_4)$alkyl, or $R^4$ represents phenylmethyloxycarbonyl and $R^3$ represents hydrogen; a group of

the formula

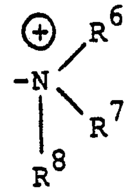

wherein $R^6$ , $R^7$ and $R^8$ each independently represent a $(C_1-C_4)$alkyl,
or $R^1$ and $R^2$ taken together with the adjacent nitrogen atom represent a saturated 5-7 membered heterocyclic ring which may optionally bear one to two $(C_1-C_4)$alkyl substituents on the ring carbons and may contain a further heterogroup selected from -O-, -S-, and $-NR^5$ - wherein $R^5$ is defined as above;
A represents hydrogen or $-N[(C_{10}-C_{11})$aliphatic acyl]beta-D-2-deoxy-2-amino-glucopyranosyl,
B represents hydrogen or N-acetyl-beta-D-2-deoxy-2-amino-glucopyranosyl,
M represents hydrogen or alpha-D-mannopyranosyl;
with the proviso that when W represents a group

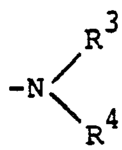 ,

a group

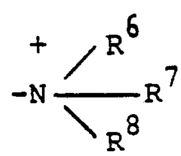 ,

ureido, guanidino or a nitrogen containing 5-6 membered heterocyclic ring as defined above directly connected with the "alk" moiety through a bond with a ring nitrogen atom, the linear alkylene "alk" moiety must be of at least two carbon atoms;
and the addition salts thereof.

A further preferred group of compounds of the invention includes those compounds of formula I wherein Y represents $NR^1R^2$, wherein $R^1$ represents hydrogen and $R^2$ represents a group -alk-W wherein "alk" is a linear alkylene chain of 2 to 8 carbon atoms, W represent pyrrolidino, morpholino, thiomorpholino, piperidino or a piperazino optionally substituted on the N′ nitrogen atom with a $(C_1-C_6)$alkyl, $(C_4-C_7)$cycloalkyl, benzyl, pyridinyl, or $(C_1-C_4)$alkylpyridinio group or W represents a group of the formula

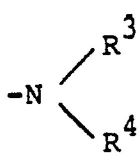

wherein $R^3$ and $R^4$ each independently represent a $(C_1-C_6)$alkyl group and A, B and M are the same as above and the acid addition salts thereof.

Also preferred compounds of the invention are represented by those compounds of formula I wherein Y represents $NR^1R^2$, wherein $R^1$, A, B and M represent hydrogen atoms and $R^2$ represents a group

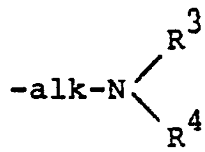

wherein "alk" is a linear alkylene chain of 2 to 6 carbon atoms and $R^3$ and $R^4$ represent ($C_1$-$C_6$)alkyl groups and the pharmaceutically acceptable addition salts thereof.

Another group of preferred compounds of the invention are those compounds of formula I wherein Y represents $NR^1$ $R^2$ and $R^1$ represents hydrogen or ($C_1$-$C_4$)alkyl, $R^2$ represents a wholly saturated nitrogen containing 5-6 membered heterocyclic ring which may contain a further N atom wherein 1 to 3 of the ring carbons may optionally bear ($C_1$-$C_4$)alkyl substituents, one of the ring nitrogens may optionally bear a substituent $R^5$ representing ($C_1$-$C_4$)alkyl and two of the ring members are bridged by an alkylene chain of 1 to 3 carbon atoms wherein one of the methylene groups may optionally be replaced by -NH- or -N[($C_1$-$C_4$)alkyl];

or a group -alk-W wherein alk represents a linear alkylene chain of 1 to 3 carbon atoms and W is a wholly saturated nitrogen containing 5-6 membered heterocyclic ring defined as in the paragraph immediately above.

Another group of preferred compounds of the invention is represented by those compounds wherein A, B, and M either represents the sugar moieties as above defined or each simultaneously represents a hydrogen atom.

Other most preferred compounds are those of formula I wherein A, B and M either simultaneously represent the sugar moieties defined above or each simultaneously represent a hydrogen atom, and Y represents $NR^1R^2$ which is in turn a group -HN(alk)W wherein "alk" represents a linear alkylene chain of 2, 3, 4, 5, 6, 7 or 8 units and W represents a group selected from: $-NH_2$, $-NHCH_3$, $-NHC_2H_5$, $-N(CH_3)_2$, $-N(C_2H_5)_2$, and $-N(CH_3)(C_2H_5)$, or a group $-HNCH(COOCH_3)(CH_2)_4NH_2$,

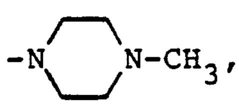 or

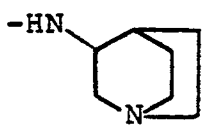

The compounds of the invention can form salts according to conventional procedures.

All the compounds of the invention can form acid addition salts.

In addition, those compounds of the invention wherein Y is hydroxy or which contain acid functions in the $-NR^1$ $R^2$ moiety may also form base addition salts.

In general, those compounds of the invention which contain acid and basic functions can form internal salts. For the scope of the present invention the "internal salts" are encompassed by the definition of the "non-salt" form.

Preferred addition salts of the compounds of this invention are the pharmaceutically acceptable acid and/or base addition salts.

With the term "pharmaceutically acceptable acid and/or base addition salts" are intended those salts with acids and/or bases which from biological, manufacturing and formulation standpoint are compatible with the pharmaceutical practice as well as with the use in the animal growth promotion.

Representative and suitable acid addition salts of the compounds of formula I include those salts formed by standard reaction with both organic and inorganic acids such as, for example, hydrochloric, hydrobromic, sulfuric, phosphoric, acetic, trifluoroacetic, trichloroacetic, succinic, citric, ascorbic, lactic, maleic, fumaric, palmitic, cholic, pamoic, mucic, glutamic, camphoric, glutaric, glycolic, phthalic, tartaric, lauric, stearic, salicylic, methanesulfonic, benzenesulfonic, sorbic, picric, benzoic, cinnamic and the like acids.

Representative examples of these bases are: alkali metal or alkaline-earth metal hydroxide such sodium, potassium, and calcium hydroxide; ammonia and organic aliphatic, alicyclic or aromatic amines such as methylamine, dimethylamine, trimethylamine, and picoline.

When the compounds of the invention contain a ($C_1$-$C_4$)alkylpyridinio or a - $\overset{\oplus}{N}R^6R^7R^8$ moiety wherein $R^6$ , $R^7$ and $R^8$ have the same meanings as above, the respective anion is an anion derived from a pharmaceutically acceptable acid. Representative examples of said anion are those deriving from the acids listed above.

The transformation of the free amino or non-salt compounds of the invention into the corresponding addition salts, and the reverse, i.e. the transformation of an addition salt of a compound of the invention into the non-salt or free amino form, are within the ordinary technical skill and are encompassed by the present invention.

For instance, a compound of formula I can be transformed into the corresponding acid or base addition-salt by dissolving the non-salt form in an aqueous solvent and adding a slight molar excess of the selected acid or base. The resulting solution or suspension is then lyophilized to recover the desired salt. Instead of lyophilizing, in some instances, it is possible to recover the final salt by extraction with an organic solvent, concentration to a small volume of the separated organic phase and precipitation by adding a non-solvent.

In case the final salt is unsoluble in an organic solvent where the non-salt form is soluble it is recovered by filtration from the organic solution of the non-salt form after addition of the stoichiometric amount or a slight molar excess of the selected acid or base.

The non-salt form can be prepared from a corresponding acid or base salt dissolved in an aqueous solvent which is then neutralized to free the non-salt form. This is then recovered for instance by extraction with an organic solvent or is transformed into another base or acid addition salt by adding the selected acid or base and working up as above.

When following the neutralization desalting is necessary, a common desalting procedure may be employed.

For example, column chromatography on controlled pore polydextrane resins (such as Sephadex L H 20) or silanized silica gel may be conveniently used. After eluting the undesired salts with an aqueous solution, the desired product is eluted by means of linear gradient or step-gradient of a mixture of water and a polar or apolar organic solvent, such as acetonitrile/water from 50:50 to about 100% acetonitrile.

As is known in the art, the salt formation either with pharmaceutically acceptable acids (bases) or non-pharmaceutically acceptable acids (bases) may be used as a convenient purification technique. After formation and isolation, the salt form of a compound of formula I can be transformed into the corresponding non-salt or into a pharmaceutically acceptable salt.

In view of the similarity of the properties of the compounds of formula I and their salts, what is said in the present application when dealing with the biological activities of the compounds of formula I applies also to their pharmaceutically acceptable salts, and viceversa.

According to the method of the invention, a teicoplanin starting compound selected from teicoplanin complex, teicoplanin $A_2$ component 1, component 2, component 3, component 4, component 5, antibiotic L 17054, antibiotic L 17046, deglucoteicoplanin or a carboxy amide or ester thereof, i.e. a compound of formula II wherein:

Y represents hydroxy, an ester function or an amide function,
A represents hydrogen or -N[($C_{10}$-$C_{11}$)aliphatic acyl]-beta-D-2-deoxy-2-amino-glucopyranosyl,
B represents hydrogen or N-acetyl-beta-D-2-deoxy-amino-glucopyranosyl,
M represents hydrogen or alpha-D-mannopyranosyl;
or an addition salt thereof,

is reacted with an alkali metal or earth alkali metal borohydride such as sodium, potassium or calcium borohydride, or sodium or potassium cyanoborohydride in the presence of a Palladium-based hydrogenation catalyst such as Palladium or a salt thereof optionally on a suitable carrier such as carbon, calcium carbonate and the like.

Also with the definition of the starting materials, the meaning Y equal to an "ester function" and an "amide function" are as defined above with reference to EP-A-216775 and EP-A- 218099.

The reaction temperature is in general between 10 °C and 60 °C, higher temperatures resulting in a faster reaction time but also in increased formation of 22,55-didechloro derivative. A most preferred reaction temperature is between 35 °C and 40 °C.

Obviously, the reaction time varies with the other parameters such as specific reactants and their concentration, temperature, etc.. In any case the reaction course may be followed by usual means, such as preferably HPLC analysis of samples at given times and, therefore, the man skilled in the art is capable of deciding when the reaction can be considered as complete and the work up procedure may be started.

When a minor amount (generally up to 10-20%) of 22,55-didechloro derivative is co-formed it can be separated by column chromatography, preferably reverse-phase partition chromatography. This compound, in fact is generally more polar than the corresponding 22-dechloro derivative.

Generally, a large excess of the borohydride and the Palladium derivative are needed.

A molar proportion of from 50 to 600 (over the teicoplanin starting material) is in general required for the borohydride, while a molar proportion of from 5 to 50 (over the teicoplanin starting material) is in general required for the Palladium derivative.

In general, a higher proportion of these reactants is required by those starting materials wherein A, B and Z represent sugar units, while a lower proportion of reactants is required by those starting materials wherein A, B and Z represent hydrogen atoms.

The same general finding holds true also for the reaction temperature and time. In fact, if the other parameters are constant, shorter reaction times or lower temperatures are required for deglucoteicoplanin derivatives than for teicoplanin $A_2$.

The reaction solvent is a polar organic solvent selected among those known and commonly used in the art.

Preferred polar organic solvents are alkanols of one to six carbon atoms, such as methanol, ethanol and propanol. Other polar organic solvents which can be preferably used in admixture with an alkanol as above defined are: dimethylformamide, diethylformamide and the like.

According to the method of the invention, teicoplanin $A_2$ component 1 is transformed into 22-dechloroteicoplanin $A_2$ component 3 since the unsaturation on the acyl chain (see the meaning of A) is reduced when removing the 22-chloro atom.

Therefore, when teicoplanin $A_2$ (complex) is used as the starting material in the process of the invention, a mixture of four main components is obtained which consists of 22-dechloroteicoplanin $A_2$ component 2, component 3, component 4 and component 5. Said mixture may be separated into its single components according to the techniques analogously known in the art (see for instance British Patent Application Publication No. 2121401). For clarity, both the mixture itself as obtained from the reaction and each of the derivatives are intended to form part of this invention as claimed here with the meaning of A representing -N[$C_{10}$-$C_{11}$)aliphatic acyl]-beta-D-2-deoxy-2-amino-glucopyranosyl. Conversely, the single pure derivatives of each teicoplanin $A_2$ component is obtained by following the process of the invention starting from the single component itself instead of starting from the complex (with the exception of what has been noted above for teicoplanin $A_2$ component 1).

Other functions that may be labile under the reaction conditions of the process of the invention, e.g. Y equal to haloalkyl, need either to be protected or masked according to known per se techniques before submitting to the dechlorination reaction of the invention or must be introduced only after this reaction is completed. The man skilled in the art is capable of finding the proper reaction condition and scheme on the basis of the present disclosure and the common knowledge in the art.

In addition, the sugar moiety of a compound of formula I may be selectively removed to transform it into another compound of formula I.

For example, a compound of formula I wherein A, B, and M represent a sugar moiety as above defined can be transformed into the corresponding compound wherein B and M are as above and A is hydrogen by means of controlled acid hydrolysis in a strong concentrated aqueous organic acid. The concentrated organic acid in this case is preferably aqueous trifluoroacetic acid at a concentration between 75% and 95%, and the reaction temperature is preferably between 10° and 50°C. The preferred hydrolysis conditions are represented by about 90% trifluoroacetic acid at room temperature. The reaction time varies depending on the other specific reaction parameters but, in any case, the reaction may be monitored by TLC or preferably HPLC techniques. An analogous selective hydrolysis is reported in European Patent Application Publication No. 146822.

Similarly, compounds of formula I wherein A, B, and M represent a sugar moiety as above defined or A represents hydrogen and B and M represent sugar moieties as above defined can be transformed into the corresponding compounds of formula I wherein A and M represent hydrogen and B represent a sugar moiety, as defined, by means of a selective hydrolysis with a strong acid in the presence of a polar aprotic solvent selected from ethers, ketones, and mixture thereof which are liquid at room temperature. Preferred hydrolysis conditions are in this case represented by the use of a concentrated mineral acid in the presence of an ether such as dimethoxyethane at room temperature. Also in this case, the reaction course may be monitored by TLC or preferably HPLC. An analogous selective hydrolysis is reported in European Patent Application Publication No. 175100.

According to another embodiment of the present invention, a compound of formula I wherein A, B and M represents sugar moieties as defined above, a compound of formula I wherein A represents hydrogen and B and M represent the above defined sugar moieties, or a compound of formula I wherein A and M represent hydrogen, and B represents a sugar moiety as above defined may be transformed into the corresponding compound of formula I wherein A, B and M represents hydrogen atoms by means of a

selective hydrolysis in an organic protic solvent selected from aliphatic acids and alpha-halogenated aliphatic acids which at the reaction temperature are liquids, aliphatic and cycloaliphatic alkanols which at the reaction temperature are liquids slightly mixable with water, phenylsubstituted lower alkanols wherein the phenyl moiety may optionally carry $(C_1-C_4)$alkyl, $(C_1-C_4)$alkoxy or halo rests which at the reaction temperature are liquids slightly mixable with water, and beta-polyhalogenated lower alkanols, which at the reaction temperature are liquids; in the presence of a strong acid, compatible with the solvent, selected from strong mineral acids, strong organic acids and strong acid cation exchange resins in the hydrogen form and at a temperature between 20° C and 100° C.

In this case, the preferred hydrolysis conditions are represented by the use of a mineral acid, such as hydrochloric acid, in an haloalkanol such as trifluoroethanol, at a temperature between 65° C and 85° C.

Analogous selective hydrolysis conditions on a similar substrate are described in European Patent Application publication No. 146053.

In the following table (Table I) the structure formulas of representative compounds of the invention are reported.

## TABLE I

| Compound | A | B | M | Y |
|---|---|---|---|---|
| 1 | $-GNHCOR_{(2-5)}$ | $-GNHCOCH_3$ | -M | $-NH(CH_2)_3N(CH_3)_2$ |
| 2 | do | do | do | $-NH(CH_2)_3N(C_2H_5)_2$ |
| 3 | $-GNHCOR_{(2)}$ | do | do | do |
| 4 | $-GNHCOR_{(2-5)}$ | do | do | $-NH(CH_2)_3N(n-C_4H_9)_2$ |
| 5 | do | do | do | $-NH(CH_2)_2-$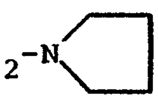 |
| 6 | do | do | do | 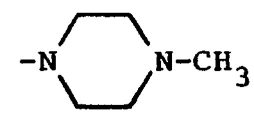 |

TABLE I (continued)

| Compound | A | B | M | Y |
|---|---|---|---|---|
| 7 | H | $-GNHCOCH_3$ | -M | $-NH(CH_2)_3N(CH_3)_2$ |
| 8 | do | do | do | $-NH(CH_2)_3N(C_2H_5)_2$ |
| 9 | do | do | do | $-NH(CH_2)_3N(n-C_4H_9)_2$ |
| 10 | do | do | do | $-NH(CH_2)_2$ 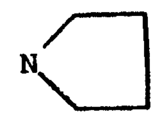 |
| 11 | do | do | do | 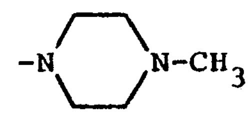 |
| 12 | do | do | H | $-NH(CH_2)_3N(CH_3)_2$ |

TABLE I (continued)

| Compound | A | B | M | Y |
|---|---|---|---|---|
| 13 | H | $-GNHCOCH_3$ | H | $-NH(CH_2)_2-N$ 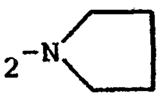 |
| 14 | do | do | do | $-NHCH_2COOC_2H_5$ |
| 15 | do | do | do | $-NHCH_2COOCH_3$ |
| 16 | do | H | do | $-NH(CH_2)_3N(CH_3)_2$ |
| 17 | do | do | do | $-NH(CH_2)_3N(C_2H_5)_2$ |
| 18 | do | do | do | $-NH(CH_2)_3N(n-C_4H_9)_2$ |
| 19 | do | do | do | $-NH(CH_2)_2-N$ 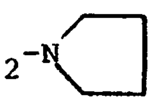 |

TABLE I (continued)

| Compound | A | B | M | Y |
|---|---|---|---|---|
| 20 | H | H | H | 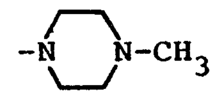 |
| 21 | $-GNHCOR_{(2-5)}$ | $-GNHCOCH_3$ | -M | $-NHCH-(CH_2)_4NH_2$<br>COOH |
| 22 | H | do | do | $-NH-CH-(CH_2)_4NH_2$<br>COOH |
| 23 | H | H | H | 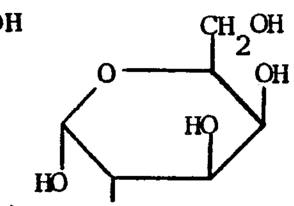<br>$-NH(CH_2)_5CO-NH$ |

TABLE I (continued)

| Compound | A | B | M | Y |
|---|---|---|---|---|
| 24 | $-GNHCOR_{(2-5)}$ | $-GNHCOCH_3$ | M | $-HN(CH_2)_3N(C_5H_{11})_2$ |
| 25 | do | do | do | $-HN(CH_2)_3N(C_6H_{13})_2$ |
| 26 | do | do | do | $-NH(CH_2)_4-CHNH_2$<br>$COOH$ |
| 27 | do | do | do | $-NH(CH_2)_4CHNH_2$<br>$COOCH_3$ |
| 28 | do | do | do | $-NHCH_2COOCH_3$ |

TABLE I (continued)

| Compound | A | B | M | Y |
|---|---|---|---|---|
| 29 | $-GNHCOR_{(2-5)}$ | $-GNHCOCH_3$ | -M | $-NHCH(CH_2)_2COOH$<br>COOH |
| 30 | do | do | do | $-NHCH(CH_2)_2CONH_2$<br>COOH |
| 31 | H | $-GNHCOCH_3$ | -M | $-NH(CH_2)_3N(C_5H_{11})_2$ |
| 32 | do | do | do | $-NH(CH_2)_3N(C_6H_{13})_2$ |
| 33 | do | do | do | $-NH(CH_2)_4-CHNH_2$<br>COOH |

TABLE I (continued)

| Compound | A | B | M | Y |
|---|---|---|---|---|
| 34 | H | $-GNHCOCH_3$ | -M | $-NH(CH_2)_4-CHNH_2$<br>$COOCH_3$ |
| 35 | do | do | do | $-NHCH_2COOCH_3$ |
| 36 | do | do | do | $-NHCH(CH_2)_2COOH$<br>$COOH$ |
| 37 | do | do | do | $-NHCH(CH_2)_2CONH_2$<br>$COOH$ |

TABLE I (continued)

| Compound | A | B | M | Y |
|---|---|---|---|---|
| 38 | H | $GNHCOCH_3$ | H | $-NHCH(CH_2)_4NH_2$<br>$\quad | $<br>$\quad COOC_2H_5$ |
| 39 | do | do | do | $-HN(CH_2)_3N(C_5H_{11})_2$ |
| 40 | do | do | do | $-HN(CH_2)_3N(C_6H_{13})_2$ |
| 41 | do | do | do | $-NH(CH_2)_4-CHNH_2$<br>$\quad | $<br>$\quad COOCH_3$ |

TABLE I (continued)

| Compound | A | B | M | Y |
|---|---|---|---|---|
| 42 | H | $-GNHCOCH_3$ | H | $-NH(CH_2)_4-CHNH_2$<br>COOH |
| 43 | do | do | do | $-NHCH_2COOH$ |
| 44 | do | do | do | $-NHCH(CH_2)_2COOH$<br>COOH |
| 45 | do | do | do | $-NHCH(CH_2)_2CONH_2$<br>COOH |

TABLE I (continued)

| Compound | A | B | M | Y |
|---|---|---|---|---|
| 46 | H | H | H | $-NH(CH_2)_3N(C_5H_{11})_2$ |
| 47 | do | do | do | $-NH(CH_2)_3N(C_6H_{13})_2$ |
| 48 | do | do | do | $-NH(CH_2)_4\underset{\underset{\textstyle COOCH_3}{\vert}}{C}HNH_2$ |
| 49 | do | do | do | $-NH(CH_2)_4\underset{\underset{\textstyle COOH}{\vert}}{C}HNH_2$ |

TABLE I (continued)

| Compound | A | B | M | Y |
|---|---|---|---|---|
| 50 | H | H | H | $-NHCH_2COOH$ |
| 51 | do | do | do | $-NHCH(CH_2)_2COOH$<br>COOH |
| 52 | do | do | do | $-NHCH(CH_2)_2CONH_2$<br>COOH |

TABLE I (continued)

| Compound | A | B | M | Y |
|---|---|---|---|---|
| 53 | $-GNHCOR_{(2-5)}$ | $-GNHCOCH_3$ | -M | 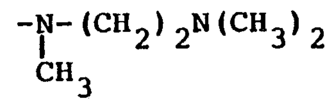 |
| 54 | $-GNHCOR_{(2,3)}$ | do | do | 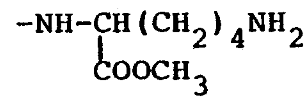 |
| 55 | H | H | H | 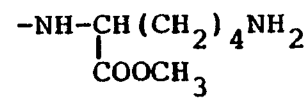 |
| 56 | $-GNHCOR_{(2-5)}$ | $-GNHCOCH_3$ | -M | $-N(CH_3)_2$ |
| 57 | do | do | do | 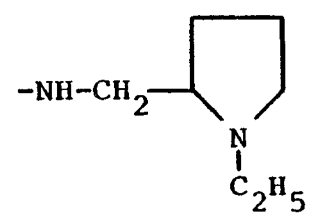 |

TABLE I (continued)

| Compound | A | B | M | Y |
|---|---|---|---|---|
| 58 | H | H | H | 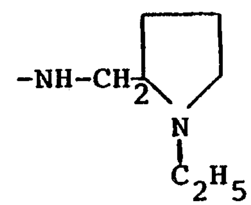 |
| 59 | $-GNHCOR_{(2-5)}$ | $-GNHCOCH_3$ | -M | $-NH(CH_2)_6NH_2$ |
| 60 | do | do | do | $-NH(CH_2)_4N(CH_3)_2$ |
| 61 | $-GNHCOR_{(2)}$ | do | do | do |

TABLE I (continued)

| Compound | A | B | M | Y |
|---|---|---|---|---|
| 62 | $-GNHCOR_{(2-5)}$ | $-GNHCOCH_3$ | -M | 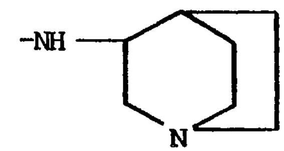 |
| 63 | H | H | H | 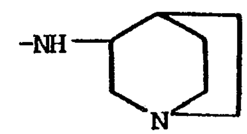 |
| 64 | do | do | do | $-NH-CH(COOH)-(CH_2)_4-NH_2$ |

TABLE I (continued)

| Compound | A | B | M | Y |
|---|---|---|---|---|
| 65 | $-GNHCOR_{(2-5)}$ | $-GNHCOCH_3$ | -M | $-NH-CH_2-COOC_2H_5$ |
| 66 | do | do | do | $-NH-CH_2-COOH$ |
| 67 | do | do | do | $-NH(CH_2)_5N(CH_3)_2$ |
| 68 | do | do | do | $-NH(CH_2)_7N(CH_3)_2$ |
| 69 | $-GNHCOR_{(2)}$ | do | do | do |

TABLE I (continued)

| Compound | A | B` | M | Y |
|---|---|---|---|---|
| 70 | $-GNHCOR_{(2-5)}$ | $-GNHCOCH_3$ | M | 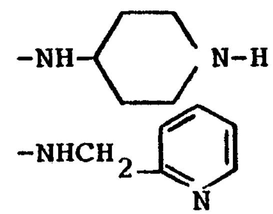 |
| 71 | do | do | do | |
| 72 | do | do | do | $-NHCH(CH_2)_3CH_3$<br>$COOCH_3$ |
| 73 | H | H | H | $-N-(CH_2)_2N(CH_3)_2$<br>$CH_3$ |
| 74 | do | do | do | $-NH(CH_2)_2N(CH_3)_2$ |
| 75 | $-GNHCOR_{(2)}$ | do | do | do |

TABLE I (continued)

| Compound | A | B | M | Y |
|---|---|---|---|---|
| 76 | $-GNHCOR_{(2-5)}$ | $-GNHCOCH_3$ | M | $-NH(CH_2)_2N(CH_3)_2$ |
| 77 | do | do | do | $-NH(CH_2)_4NH_2$ |
| 78 | H | H | H | $-NH(CH_2)_4N(CH_3)_2$ |
| 79 | do | do | do | $-NH(CH_2)_4NH_2$ |

TABLE I (continued)

| Compound | A | B | M | Y |
|---|---|---|---|---|
| 80 | H | H | H | $-NH(CH_2)_5N(CH_3)_2$ |
| 81 | do | do | do | $-NH(CH_2)_7N(CH_3)_2$ |
| 82 | do | do | do | $-NH(CH_2)_6NH_2$ |
| 83 | $-GNHCOR_{(2-5)}$ | $-GNHCOCH_3$ | -M | OH |
| 83a | $-GNHCOR_{(2,3)}$ | do | do | OH |

TABLE I (continued)

| Compound | A | B | M | Y |
|---|---|---|---|---|
| 84 | $-GNHCOR_{(2,3)}$ | $-GNHCOCH_3$ | H | OH |
| 85 | $-GNHCOR_{(2)}$ | do | -M | OH |
| 86 | $-GNHCOR_{(2-5)}$ | H | H | OH |
| 87 | H | H | H | OH |
| 88 | H | $-GNHCOCH_3$ | -M | OH |

**TABLE I** (continued)

| Compound | A | B | M | Y |
|---|---|---|---|---|
| 89 | H | $-GNHCOCH_3$ | H | OH |
| 90 | $-GNHCOR_{(2)}$ | $-GNHCOCH_3$ | -M | OH |
| 91 | $-GNHCOR_{(2-5)}$ | $-GNHCOCH_3$ | -M | $-OCH_2CH_3$ |
| 92 | H | H | H | $-OCH_2CH_2CH_2CH_3$ |

**TABLE I (continued)**

| Compound | A | B | M | Y |
|---|---|---|---|---|
| 93 | $-GNHCOR_{(2-5)}$ | $-GNHCOCH_3$ | -M | $-OCH_2CH_2CH_2CH_2CH_3$ |
| 94 | do | do | H | $-OC_2H_5$ |
| 95 | $-GNHCOR_{(2)}$ | do | -M | $-OCH_2CH_2CH_2CH_3$ |
| 96 | $-GNHCOR_{(2-5)}$ | $-GNHCOCH_3$ | do | $-OCH_2CH(CH_3)_2$ |

TABLE I (continued)

| Compound | A | B | M | Y |
|---|---|---|---|---|
| 97 | H | H | H | $-OCH_2CH_2CH_3$ |
| 98 | H | $-GNHCOCH_3$ | -M | $-OCH_3$ |
| 99 | H | $-GNHCOCH_3$ | H | $-OCH_2CH(CH_3)_2$ |
| 100 | $-GNHCOR_{(2)}$ | $-GNHCOCH_3$ | -M | $-OCH_2CH_2CH_2CH_2CH_2CH_3$ |

Note:

$-GNHCOR_{(2-5)}$ = N/(C$_{10}$-C$_{11}$)aliphatic acyl/-beta-D-2-deoxy-2-aminoglucopyranosyl

$-GNHCOR_{(2,3)}$ = N-(8-methylnonanoyl)-beta-D-2-deoxy-2-aminoglucopyranosyl and N-decanoyl-beta-D-2-deoxy-2-aminoglucopyranosyl

$-GNHCOCH_3$ = N-acetyl-beta-D-2-deoxy-2-aminoglucopyranosyl

-M = alpha-D-mannopyranosyl

The following Table (Table II) reports the analytical data of some representative compounds of the invention, while Table IIa reports, for comparison purposes, the analytical data of the 22,55-didechloro derivatives of the compounds reported in Table II.

Table II

Summary of the analytical data of 22-dechloro derivatives of teicoplanin

| Compound | HPLC[a] ($t_R$, min) | FAB-MS ($MH^+$) | Cl% (Found/Calcd) | Formula |
|---|---|---|---|---|
| 83 | 15.7 | n.d. | 1.95/1.92 | --- |
| 88 | 9.5 | 1530 | 2.08/2.31 | $C_{72}H_{69}O_{28}N_8Cl$ |
| 89 | 10.5 | 1368 | 2.46/2.59 | $C_{66}H_{59}O_{23}N_8Cl$ |
| 87 | 12 | 1164 | 2.5/3.04 | $C_{58}H_{46}O_{18}N_7Cl$ |
| 55 | n.d. | 1306 | 2.53/2.71 | $C_{65}H_{60}O_{19}N_9Cl$ |
| 92 | 11.5[b] | n.d. | 2.83/2.9 | $C_{62}H_{54}O_{18}N_7Cl$ |
| 97 | 10.4[b] | n.d. | 2.86/2.93 | $C_{61}H_{52}O_{18}N_7Cl$ |

a = Linear gradient from 5 to 75% of $CH_3CN$ in 0.2% aq. $HCO_2CH_4$ in 35 min at the flow rate of 2 mL/min.

b = Linear gradient from 20 to 75% of $CH_3CN$ in 0.2% aq. $HCO_2CH_4$ in 35 min at the flow rate of 2 mL/min.

Table IIa

Summary of the analytical data of 22,55-didechloro derivatives corresponding to 22-dechloro derivatives reported in Table II (the number into brackets refers to the number of the corresponding mono-chloro derivative)

| Compound * | HPLC[a] ($t_R$, min) | FAB-MS ($MH^+$) | Cl% (Found/Calcd) | Formula |
|---|---|---|---|---|
| (83) | 14.5 | n.d. | 0.05/- | --- |
| (88) | 8.5 | 1495 | 0.11/- | $C_{72}H_{70}O_{28}N_8$ |
| (89) | 10 | 1333 | 0.12/- | $C_{66}H_{60}O_{28}N_8$ |
| (87) | 11 | 1129 | 0.4/- | $C_{58}H_{47}O_{18}N_7$ |
| (55) | n.d. | 1283 | 0.06/- | $C_{65}H_{61}O_{19}N_9$ |
| (92) | 10[b] | n.d. | 0.07/- | $C_{62}H_{55}O_{18}N_7$ |

* = Didechloro compound corresponding to the mono-chloro compound reported above

a = Linear gradient from 5 to 75% of $CH_3CN$ in 0.2% aq. $HCO_2CH_4$ in 35 min at the flow rate of 2 mL/min.

b = Linear gradient from 20 to 75% of $CH_3CN$ in 0.2% aq. $HCO_2CH_4$ in 35 min at the flow rate of 2 mL/min.

The antibacterial activity of the compounds of the invention can be demonstrated in vitro by means of standard agar-dilution tests.

Isosensitest broth (Oxoid) and Todd-Hewitt broth (Difco) are used for growing staphylococci and streptococci, respectively. Broth cultures are diluted so that the final inoculum is about $10^4$ colony forming units/ml (CFU/ml). Minimal inhibitory concentration (MIC) is considered as the lowest concentration which shows no visible growth after 18-24 h incubation at 37° C. The results of the in vitro antibacterial testing of representative compounds of formula I are summarized in Table III, below:

TABLE III

| M.I.C. (microgram/ml) | | | | | | | |
|---|---|---|---|---|---|---|---|
| Strain | Compound | | | | | | |
| | 83 | 88 | 89 | 87 | 55 | 92 | 97 |
| Staph. aureus L165 | 1 | 0.5 | 0.25 | 0.12 | 0.12 | 0.12 | 0.12 |
| Staph. epidermidis L147 ATCC12228 | 2 | 2 | 0.25 | 0.12 | 0.06 | 0.06 | 0.06 |
| Staph. haemolyticus L602 | 8 | 32 | 4 | 1 | n.d. | 0.5 | 0.5 |
| Strep. pyogenes L49 C203 | 0.12 | 1 | 1 | 0.12 | 0.12 | 0.12 | 0.12 |
| Strep. faecalis L149 ATCC 7080 | 0.25 | 2 | 2 | 0.25 | 0.12 | 0.25 | 0.12 |
| Escherichia coli L47 SKF 12140 | >128 | >128 | >128 | 128 | 8 | 64 | 32 |

In view of the above reported antimicrobial activity, the compounds of the present invention can effectively be employed as the active ingredient of antimicrobial preparations used in human and veterinary medicine for the prevention and treatment of infectious diseases caused by pathogenic bacteria which are susceptible to said active ingredients.

In such treatments, these compounds may be employed as such or in the form of mixtures in any proportion.

The compounds of the present invention can be administered orally, topically or parenterally wherein however, the parenteral administration is preferred. Depending on the route of administration, these compounds can be formulated into various dosage forms. Preparations for oral administration may be in the form of capsules, tablets, liquid solutions or suspensions. As known in the art the capsules and tablets may contain in addition to the active ingredient, conventional excipients such as diluents, e.g. lactose, calcium phosphate, sorbitol and the like, lubricants, e.g. magnesium stearate, talc, polyethylene glycol, binding agents, e.g. polyvinylpyrrolidone, gelatin, sorbitol, tragacanth, acacia, flavoring agents, and acceptable disintegrating and wetting agents. The liquid preparations generally in the form of aqueous or oily solutions or suspensions, may contain conventional additives such as suspending agents. For topical use the compounds of the present invention may also be prepared in suitable forms for absorption through the mucous membranes of the nose and throat or bronchial tissues and may conveniently take the form of liquid sprays or inhalants, lozenges, or throat paints.

For medication of the eyes or ears, the preparation may be presented in liquid or semi-liquid form. Topical applications may be formulated in hydrophobic or hydrophilic bases as ointments, creams, lotions, paints, or powders.

For rectal administration the compounds of the invention are administered in the form of suppositories admixed with conventional vehicles, such as, for example, cocoa butter, wax, spermaceti or polyethylenglycols and their derivatives.

Compositions for injection may take such forms as suspensions, solutions, or emulsions in oily or aqueous vehicles, and may contain formulatory agents such as suspending, stabilizing and/or dispersing agents.

Alternatively, the active ingredient may be in powder form for reconstitution at the time of delivery with a suitable vehicle, such as sterile water.

The amount of active principle to be administered depends on various factors such as the size and conditions of the subject to be treated, the route and frequency of administration, and the causative agent involved.

The compound of the invention are generally effective at a dosage comprised between about 0.5 and about 30 mg of active ingredient per Kg of body weight, preferably divided in 2 to 4 administrations per day. Particularly desirable compositions are those prepared in the form of dosage units containing from

about 20 to about 300 mg per unit.

Representative examples of preparation of pharmaceutical compositions are as follows:

A parenteral solution is prepared with 100 mg of compound of Example 1 dissolved in 2 ml of sterile water for injection.

A parenteral solution is prepared with 250 mg of compound of Example 1 hydrochloride dissolved in 3 ml of sterile water for injection.

A topical ointment is prepared with 200 mg of compound of Example 1

3.6 g of polyethylene glycol 4000 U.S.P.

6.2 g of polyethylene glycol 400 U.S.P.

Besides their activity as medicaments, the compounds of the present invention can be used as animal growth promoters.

For this purpose, one or more of the compounds of the invention is administered orally in a suitable feed. The exact concentration employed is that which is required to provide for the active agent in a growth promotant effective amount when normal amounts of feed are consumed.

The addition of the active compounds of the invention to animal feed is preferably accomplished by preparing an appropriate feed premix containing the active compounds in an effective amount and incorporating the premix into the complete ration.

Alternatively, an intermediate concentrate or feed supplement containing the active ingredient can be blended into the feed.

The way in which such feed premixes and complete rations can be prepared and administered are described in reference books (such as "Applied Animal Nutrition", W.H. Freedman and Co., S. Francisco, USA, 1969 or "Livestock Feeds and Feeding", O and B Books, Corvallis, Oregon, USA, 1977) and are incorporated herein by reference.

The following examples further illustrate the invention and must not be construed as limiting it.

Example 1:

Preparation of 22-dechloroteicoplanin (Compound 83)

To a stirred suspension of 10 g (about 5 mmol) of teicoplanin (HPLC composition: teicoplanin $A_2$ component 1: 13.1%; teicoplanin $A_2$ component 2: 38.6%; teicoplanin $A_2$ component 3: 19.3%; teicoplanin $A_2$ component 4: 9.2%; teicoplanin $A_2$ component 5: 9.5%; teicoplanin $A_3$ factor 1: 10.4%) in 1 L of absolute methanol, 20 g (about 112 mmol) of $PdCl_2$ is added in one portion at room temperature under $N_2$ atmosphere. After 1 h, the reaction mixture is cooled to 0-5° C and 80 g (about 2.1 mol) of $NaBH_4$ (pellets) is added portionwise in 1 h, while maintaining the temperature below 15° C. Then the temperature is slowly brought to 35-38° C under vigorous stirring. After 8 h at 35-40° C, the reaction mixture is cooled to room temperature and stirring is continued overnight. The precipitate (elemental Palladium) is filtered off and washed twice with 150 ml of $CH_3OH$. The filtrate is adjusted to pH 5 with glacial acetic acid and then concentrated to a small volume. The precipitate which thus forms is collected by filtration (about 90 g of crude brown material) and redissolved in 600 ml of $H_2O$. This solution is applied to a silanized silica gel column (2.5 kg; 0.06-0.2 mm; Merck Co.) in 2% aqueous $HCO_2NH_4$. Desalting is performed by eluting with 1 L each of the following mixtures of $CH_3CN:H_2O$ (v/v): 1) 10:90; 2) 20:80; 3) 30:70; 4) 40:60 and 5) 50:50. Afterwards the column is developed with a solution of $CH_3CN$:0.1N HCl, 50:50 (v/v) collecting 500 ml fractions which are analyzed by HPLC. Those fractions containing 22-dechloroteicoplanin are combined, n-$C_4H_9OH$ is added and the mixture is concentrated at 25° C to obtain a dry butanolic suspension of about 250 ml. By adding ether a solid separates which is collected by filtration, washed with ether and dried in vacuo at room temperature overnight, yielding 7.5 g of the 22-dechloroteicoplanin (HPLC composition: teicoplanin $A_2$ component 1: absent; teicoplanin $A_2$ component 2: 38.9%; teicoplanin $A_2$ component 3: 32.1%; teicoplanin $A_2$ component 4: 9.1%; teicoplanin $A_2$ component 5: 9.7%; teicoplanin $A_3$ factor 1: 10.2%) as the hydrochloride.

By following the above procedure but starting from the pure components of teicoplanin, the corresponding pure 22-dechloro derivatives are obtained substantially with the same yields. Alternatively, instead of collecting the whole 22-dechloroteicoplanin component containing chromatography fractions (see the above method) only those chromatographic fractions are separately pooled which contain the same 22-dechloroteicoplanin component.

According to any of these procedures 22-dechloroteicoplanin $A_2$, component 2, 3, 4 or 5 are separately

obtained in a pure form. Teicoplanin $A_2$ component 1 on the contrary, is transformed, almost quantitatively, in 22-dechloroteicoplanin $A_2$ component 3.

The following compounds (see Table I) are prepared according to the procedure of Example 1 by using the corresponding starting materials:
Compounds: 1-6, 21, 24-30, 53, 54, 56, 57, 59-62, 65-72, 75-77, 83-86, 90, 91, 93 and 100.

Example 2:

Preparation of 22-dechloro antibiotic L 17054 (compound 88), 22-dechloro antibiotic L 17046 (Compound 89) or 22-dechlorodeglucoteicoplanin (Compound 87)

Substantially following the procedure described above but starting from 5 mmol of antibiotic L 17054, antibiotic L 17046 or deglucoteicoplanin, the corresponding 22-dechloro derivatives are obtained.

The main variations of the above method are the following:
The reactions are respectively allowed to proceed at 40 °C (internal temperature) for 8 h (antibiotic L 17054) 5 h (antibiotic L 17046) and 2 h (deglucoteicoplanin) after the end of the addition of $NaBH_4$, and the crude products thus obtained are purified by reverse-phase column chromatography using 1.4 kg of silanized silica-gel (0.06-0.2 mm; Merck Co.) in 0.2% aq. $HCO_2NH_4$ as the stationary phase, and a linear gradient from 10 to 50% of $CH_3CN$ in $H_2O$, in 10 h at the rate of 500 ml/h as the mobile phase, while collecting 25 ml fractions. Those fractions which contain the pure title compounds are pooled, an excess of n-$C_4H_9OH$ and 1N HCl is added, then the resulting mixtures are concentrated to a small volume. Depending on the starting material, by adding ether, 22-dechloro antibiotic L 17054 (2.9 mmol), 22-dechloro antibiotic L 17046 (1.8 mmol) or 22-dechlorodeglucoteicoplanin (3.5 mmol) is obtained.

For an easy reference they are indicated in the Tables reporting the analytical data as compounds of Example 88, 89 and 87, respectively.

The following compounds (see Table I) are prepared according to the procedure of Example 2 by using the corresponding starting materials:
Compounds: 7-20, 22, 23, 31-52, 55, 58, 63, 64, 73, 74, 78-82, 87-89, 92 and 97-99.

Example 3:

Preparation of the amide of 22-dechlorodeglucoteicoplanin with alpha-lysine-methyl ester (Compound 55)

To a stirred solution of alpha-lysine-methyl ester deglucoteicoplanin amide (prepared as described in EP-A-218099) (6 g; about 3.6 mmol) in 800 ml of absolute $CH_3OH$, 3.8 g of $PdCl_2$ (21.6 mmol) is added under stirring at room temperature under $N_2$ atmosphere. After 1h, the reaction mixture is cooled to 0-5 °C and 12.6 g of $NaBH_4$ (pellets) (about 360 mmol) is added over a period of 30 min while maintaining the temperature below 15 °C. After 30 min, the temperature is slowly increased to 38 °C and after 4h, the reaction is allowed to cool to room temperature. The elemental Pd which precipitates is filtered off and washed three times with 150 ml of absolute $CH_3OH$. The methanol solution is adjusted to pH 5 by adding glacial acetic acid, then the solvents are evaporated to give 30 g of a brownish residue. This crude material is purified by column chromatography on silanized silica gel (1.4 kg; 0.06-0.2 mm; Merck Co.) prepared in water and developed with a linear gradient from 10 to 60% of $CH_3CN$ in 0.01N HCl in 20 h at the rate of 400 ml/h, while collecting 20 ml fractions. Those fractions which contain the compound of the title are pooled and worked up as described in Example 1, obtaining 1.8 g of the 22-dechlorodeglucoteicoplanin amide of the title.

By following substantially the same procedure but starting from the N epsilon-carbobenzoxy derivative of the starting material, the product of the title is obtained substantially with the same yields.

Example 4:

Preparation of 22-dechloroteicoplanin aglycon, n-butyl ester (Compound 92)

To a stirred solution of 3.2 g (about 2.4 mmol) of deglucoteicoplanin n-butyl ester (prepared as described in EP-A- 216775) in 300 ml of absolute $CH_3OH$, 35 g (about 200 mmol) of $PdCl_2$ is added at room temperature, under $N_2$ atmosphere. After 45 min, 10 g (about 285 mmol) of $NaBH_4$ (pellets) is added portionwise, over a period of 1 h. The temperature is allowed to rise up to 30°C and maintained at about 30-35°C for 3h. The reaction mixture is cooled to room temperature and diluted to 500 ml with absolute $CH_3OH$. The elemental Pd which forms is filtered off through a filter aid (Celite) panel and washed 3 times with 50 ml of absolute methanol. The solution is brought to pH 5 by adding glacial acetic acid and concentrated to give 9 g of a dark brown residue which is dissolved in 100 ml of a mixture $CH_3OH:H_2O$ 3:7 (v/v). The resulting dark solution is loaded on a short column (0.06-0.2 mm; Merck Co.) of 600 g of silanized silica-gel in $H_2O$.

Desalting is performed by eluting with 1 L each of the following mixtures of $CH_3OH:H_2O$ (v/v): 1) 30:70; 2) 40:60; 3) 50:50, while collecting 500 ml fractions which are checked by HPLC. Those fractions containing the product of the title are combined, n-BuOH is added and the solvents are evaporated at 35°C, to give 1.3 g of a crude residue which was dissolved in 250 ml of a mixture of $CH_3CN$:0.01N HCl, 75:25 (v/v). The resulting solution is loaded on a column of 200 g of silanized silica-gel in $H_2O$. This column is developed with a linear gradient from 25% of $CH_3CN$ in 0.01N HCl to 75% of $CH_3CN$ in 0.1N HCl in 20 h, at the rate of 400 ml/h. Fractions of 20 ml are collected and those containing the desired product are worked up substantially as described in Example 1, yielding 1 g of the hydrochloride of the compound of the title.

Compound 97 is obtained with the same yields by following the above procedure but starting from deglucoteicoplanin n-propyl ester.

Example 5:

Conversion of 22-dechloroteicoplanin into 22-dechloro antibiotic L 17054

A solution of 2 mmol of 22-dechloroteicoplanin (see Example 1) in 160 ml of 90% aq. $CF_3COOH$ is stirred at room temperature for 90 min. The solvent is then evaporated at 40°C and the oily residue is re-dissolved in 200 ml of a mixture $CH_3CN:H_2O$ 1:1 (v/v). The organic solvent is evaporated at room temperature ad the resulting cloudy aqueous solution is brought to pH 6.5 with 0.1N NaOH. The precipitate is collected, washed with 100 ml of $H_2O$, then re-dissolved in 200 ml of a mixture $CH_3OH$:n-$C_4H_9OH$:0.1N HCl 4:4:2 (v/v/v). The resulting solution is concentrated at 45°C to a small volume (about 10 ml) and 100 ml of ether is added. The precipitate is collected, washed with ether and dried in vacuo at 50°C overnight (over KOH pellets), yielding 1.72 or 1.85 mmol of 22-dechloro antibiotic L 17054 as the hydrochloride.

Example 6:

Conversion of 22-dechloroteicoplanin or 22-dechloro antibiotic L 17054 into 22-dechloro antibiotic L 17046

Dry HCl is bubbled at room temperature into a stirred suspension of 1 mmol of 22-dechloroteicoplanin (see Example 1) or 22-dechloro antibiotic L 17054 (see Example 2), in 100 ml of 1,2-dimethoxyethane (DME) for 3 h. The solution which forms is evaporated at 50°C and the residue is re-dissolved in 100 ml of a mixture $CH_3OH$:n-$C_4H_9OH$:0.1N HCl 4:4:2 (v/v/v). Working up of the resulting solutions as described in Example 5 yields 0.6 or 0.5 mmol of the compound of the title.

Example 7:

Conversion of 22-dechloroteicoplanin, 22-dechloro antibiotic L 17054 or 22-dechloro antibiotic L 17046 into 22-dechlorodeglucoteicoplanin

A stirred suspension of 2 mmol of 22-dechloroteicoplanin, 22-dechloro antibiotic L 17054, 22-dechloro antibiotic L 17046 or a mixture thereof in 200 ml of 2,2,2-trifluoroethanol (TFE) is heated at 70°C while bubbling dry HCl. After 6 h, the insoluble is collected and dissolved in 100 ml of a mixture $CH_3CN:H_2O$ 1:1

(v/v). After adding 20 g of silanized silica-gel, the resulting suspension is diluted with 400 ml of $H_2O$ under vigorous stirring and the pH is adjusted to 5.5 with 1N NaOH. The mixture is loaded on a chromatographic column of 400 g of silanized silica-gel in $H_2O$ and the elution is carried out with a linear gradient from 10 to 50% of $CH_3CN$ in 0.001N HCl in 20 h at the rate of 200 ml/h, while collecting 15 ml fractions. Those fractions containing the desired product (HPLC) are pooled, n-$C_4H_9OH$ is added and the resulting mixture is concentrated to a small volume (about 50 ml) obtaining a cloudy dry butanolic solution. By adding 250 ml of ethyl acetate, a solid separates which is collected, washed with ether and dried in the air overnight, yielding 1.1 or 1.3 mmol of 22-dechlorodeglucoteicoplanin as the hydrochloride.

Example 8:

Hydrogenation with 10% Pd/C (c.f. Harris C.M. et al., J. Am. Chem. Soc. 107, 1985, 6652-6658)

a) From teicoplanin

A solution of 10 g (about 5 mmol) of teicoplanin (c.f. Example 1) in 500 ml of a mixture $CH_3OH:H_2O$ 7:3 (v/v) is adjusted to pH 7.6 with 0.01N NaOH and hydrogenated (in a Parr apparatus) at room temperature and pressure in the presence of 5 g of 10% Pd/C over a period of 6 h. A suspension of 10 g of the same catalyst in 200 ml of $H_2O$ is added and hydrogenation continued at 4 atm for 6 h. The dark suspension is then brought to pH 2.5 with 1N HCl and the catalyst is removed by filtration through a panel of 50 g of celite BDH-545 filter-aid. The clear filtrate is brought to pH 5.6 with 1N NaOH and concentrated in the presence of 50 ml of n-$C_4H_9OH$ to eliminate most of the $CH_3OH$. By adding 100 ml of 1% aq. $HCO_2NH_4$ a solid separates from the cloudy aqueous solution, which is collected, washed with 100 ml of $H_2O$ and dried in vacuo at 40°C for 3 days, yielding 6.4 g of 22,55-didechloroteicoplanin as the internal salt.

A suspension of 2 g of this product in 100 ml of $CH_3OH$ is stirred while adding 0.12 ml of 37% aq. HCl followed by 500 ml of ethyl acetate. The precipitate is collected, washed with 100 ml of ether, then dried in vacuo at room temperature over $P_2O_5$, for 3 days yielding 1.6 g of the above compound, as the hydrochloride.

b) From antibiotic L 17054, antibiotic L 17046 or deglucoteicoplanin

By following the above procedure with the variations indicated below 3.6 mmol of 22,55-didechloro antibiotic L 17054, 3.4 mmol of 22,55-didechloro antibiotic L 17046 or 2.9 mmol of 22,55-didechlorodeglucoteicoplanin are obtained starting from 5 mmol of antibiotic L 17054, antibiotic L 17046 or deglucoteicoplanin. The starting material in 500 ml of a mixture $CH_3OH$:0.04N HCl 7:3 (v/v) is hydrogenated at room temperature and at 1 atm (98 MPa) for 3 h over 5 g of 10% Pd/C, then at 4 atm over 10 g of the same catalyst for 3, 2.5 and 2 h, respectively. After filtration and evaporation of the solvents, the above mentioned products, depending on the starting materials, are obtained as the hydrochloride, without further purification.

c) From alpha-lysine methyl ester deglucoteicoplanin amide

c.1) A solution of 14 g (about 10 mmol) of the amide of the 63-carboxy group of deglucoteicoplanin with the alpha-amino group of lysine methyl ester (see EP-A-218099 and Example 3) in 700 ml of a mixture $CH_3OH$:0.04N HCl 7:3 (v/v) is hydrogenated at room temperature and pressure for 6 h in the presence of 10 g of 10% Pd/C, while absorbing 630 ml of $H_2$. HPLC analysis of this reaction mass shows unreacted starting material (about 20%), 22-dechloro derivative (about 45%) and 22,55-didechloro derivative (about 35%). After adding additional 5 g of the same catalyst, hydrogenation is continued for 1 h under the above conditions (during this period about 240 ml of $H_2$ is absorbed). The catalyst is then filtered off and 50 g of silanized silica-gel and 500 ml of n-butanol are added to the filtrate (HPLC analysis: starting material absent, 22-dechloro derivative about 60%, 22,55-didechloro derivative about 40%). The resulting suspension is evaporated to dryness at 60°C. The residue is applied to a chromatographic column of 1.4 kg of silanized silica-gel in $H_2O$. Development of the column with a linear gradient from 10 to 60% of $CH_3CN$ in 0.01N HCl

in 20 h at the rate of 400 mL/h, while collecting 20 ml fractions, and working up of those fractions which contain the compounds of the title, yields 4.1 g of 22-dechloro derivative and 2.6 g of 22,55-didechloro derivative as the hydrochlorides.

c.2) A solution of 10 mmol of the amide obtained from condensation of the 63-carboxy group of deglucoteicoplanin and the alpha-amino group of lysine methyl ester (see above) in 1.5 L of a mixture $CH_3OH$:0.01N HCl 8:2 (v/v) is hydrogenated at room temperature and pressure in the presence of 5 g of 10% Pd/C for 2 h. Then 10 g of the same catalyst is added and hydrogenation is continued at 4 atm for 6 h. The catalyst is removed and the filtrate is brought to pH 8.5 with 1N NaOH, and concentrated at 25 °C to give an aqueous solution which was extracted with 600 ml of n-$C_4H_9OH$. The butanolic layer is washed with $H_2O$ (2 x 300 ml) and concentrated to a small volume (about 100 ml). After adding 1 ml of 37% HCl and 400 ml of ether, the precipitate which separates is collected, washed several times with ether and dried in vacuo at 50 °C overnight, yielding 7.8 g of 22,55-didechloro derivative as the hydrochloride.

Example 9:

Hydrogenation with 5% Pd/C

A solution of 10 mmol of teicoplanin, antibiotic L 17054, antibiotic L 17046 or deglucoteicoplanin, in 1 L of a mixture $CH_3OH$:0.04N HCl 7:3 (v/v) was hydrogenated at room temperature and pressure in the presence of 10 g of 5% Pd/C. Within 1-2 h, the theoretical amount of $H_2$, as calculated for the first dechlorination step (about 220 ml), was adsorbed but no transformation of the starting material was shown by HPLC. Fresh catalyst, 10 g of 5% Pd/C, was then added and hydrogenation continued for 4-5 h while observing the absorption of 200-250 ml of $H_2$ and the simultaneous formation (HPLC) of mono- and didechloro derivatives still in the presence of the starting teicoplanin compound (40-50%). After adding 10 g of 10% Pd/C, the hydrogenation was allowed to proceed to adsorb additional 250 ml of $H_2$, then the obtained dark suspension was filtered and the filtrate adjusted at pH 7.2 with 1N NaOH. Evaporation of the solvents yielded different mixtures of mono- and di-dechlorinated derivatives, depending on the proper starting material, which was in general absent. By reverse-phase column chromatography on silanized silica-gel (eluted with a linear gradient from 10% of $CH_3CN$ in 0.2% aq. $HCO_2NH_4$ to 70% of $CH_3CN$ in $H_2O$) the monodechloro teicoplanin derivative was separated from the corresponding di-dechlorinated compound. The final products were obtained by precipitation of their internal salts from aqueous solutions adjusted to pH 6-6.5.

## Claims

1. A teicoplanin derivative of formula

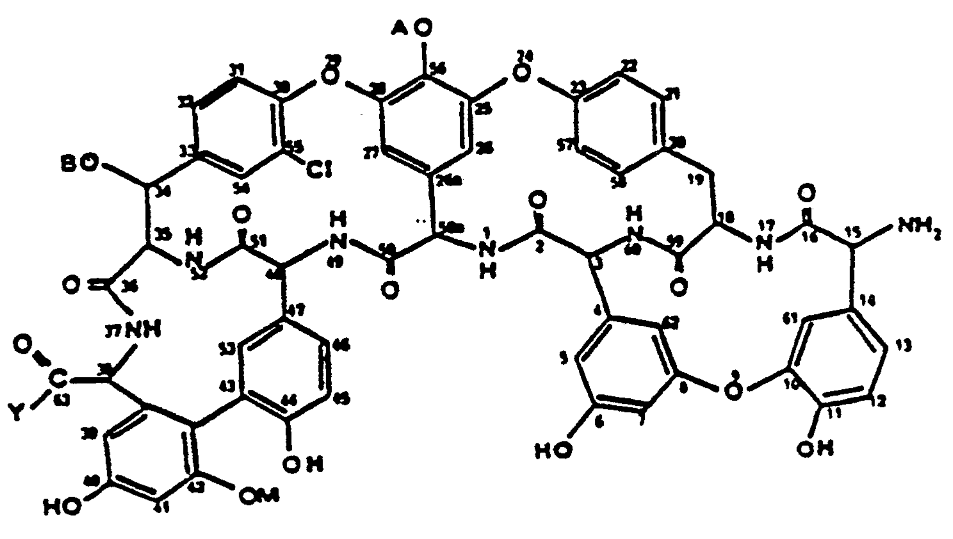

I

wherein:

Y represents hydroxy, a group $NR^1 R^2$ wherein

$R^1$ represents hydrogen, $(C_1-C_6)$alkyl, hydroxy$(C_2-C_4)$alkyl, halogeno$(C_2-C_4)$alkyl, $(C_1-C_4)$alkoxy$(C_2-C_4)$-alkyl, amino$(C_2-C_4)$alkyl, $(C_1-C_4)$alkylamino$(C_2-C_4)$alkyl, di$(C_1-C_4)$alkylamino$(C_2-C_4)$alkyl

$R^2$ represents hydrogen, $(C_1-C_6)$alkyl, hydroxy$(C_2-C_4)$alkyl, halogeno$(C_2-C_4)$alkyl, $(C_1-C_4)$alkoxy$(C_2-C_4)$-alkyl, a nitrogen containing 5-6 membered heterocyclic ring

which may be unsaturated, partially saturated or wholly saturated and may contain 1 to 3 further heteroatoms selected from N, S and O wherein 1 to 3 of the ring carbons may optionally bear $(C_1-C_4)$alkyl substituents and one of the ring nitrogens may optionally bear a substituent $R^5$ selected from $(C_1-C_4)$alkyl, $(C_4-C_7)$cycloalkyl, phenyl optionally substituted with halogen or $(C_1-C_4)$alkyl, phenyl$(C_1-C_4)$alkyl, pyridyl, $(C_1-C_4)$alkylpyridinio, and when the ring is wholly saturated two of the ring members may optionally be bridged by an alkylene chain of 1 to 3 carbon atoms wherein one of the methylene groups may optionally be replaced by -NH- or - N [$(C_1-C_4)$alkyl];

a group -alk-W wherein "alk" represents a linear alkylene chain of 1 to 8 carbon atoms which is optionally substituted with a substituent selected from $(C_1-C_4)$alkyl, hydroxy$(C_1-C_4)$alkyl, hydroxy, carboxy, aminocarbonyl, $(C_1-C_4)$alkylaminocarbonyl, di$(C_1-C_4)$alkylaminocarbonyl, $(C_1-C_4)$alkoxycarbonyl, phenyl$(C_1-C_4)$-alkoxycarbonyl, and W represents a carboxy, $(C_1-C_4)$alkoxycarbonyl, phenyl$(C_1-C_4)$alkoxycarbonyl, aminocarbonyl, $(C_1-C_4)$aminocarbonyl, di$(C_1-C_4)$aminocarbonyl, pentosaminocarbonyl, hexosaminocarbonyl, ureido, guanidino, a nitrogen containing 5-6 membered heterocyclic ring defined as above, a group of the formula

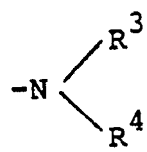

wherein $R^3$ and $R^4$ each independently represent hydrogen, $(C_1-C_6)$alkyl, hydroxy$(C_2-C_4)$alkyl and halogeno$(C_2-C_4)$alkyl, or $R^4$ represents phenylmethyloxycarbonyl and $R^3$ represents hydrogen; a group of the formula

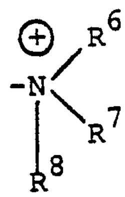

wherein $R^6$ , $R^7$ and $R^8$ each independently represent a $(C_1-C_4)$alkyl,

or $R^1$ and $R^2$ taken together with the adjacent nitrogen atom represent a saturated 5-7 membered heterocyclic ring which may optionally bear one to two $(C_1-C_4)$alkyl substituents on the ring carbons and may contain a further heterogroup selected from -O-, -S-, and $-NR^5-$ wherein $R^5$ is defined as above;

with the proviso that when W represents

a group

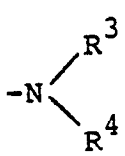 ,

a group

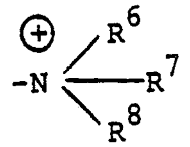 ,

ureido, guanidino or a nitrogen containing 5-6 membered heterocyclic ring as defined above directly connected with the "alk" moiety through a bond with a ring nitrogen atom, the linear alkylene "alk" moiety must be of at least two carbon atoms; or

Y represents -OR wherein R represents $(C_1-C_{12})$alkyl, hydroxy$(C_1-C_{12})$alkyl, $(C_1-C_3)$alkoxy$(C_1-C_{12})$alkyl, halo$(C_1-C_{12})$alkyl; a group of formula

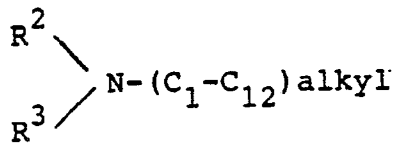

wherein $R^2$ and $R^3$ each independently represents hydrogen or $(C_1-C_4)$alkyl groups, or $R^2$ and $R^3$ taken together with the adjacent nitrogen atom represent a 5-7 membered aromatic, partially hydrogenated or saturated heterocycle ring which may optionally contain a further heteroatom selected from S, O and N; a group of formula

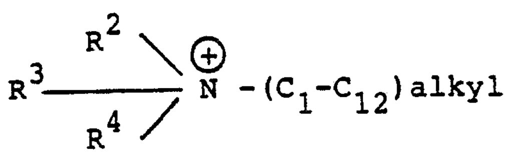

wherein $R^2$ and $R^3$ are as defined above and $R^4$ represents hydrogen or $(C_1-C_4)$alkyl; or R represents a group of formula

$H-[O(CH_2)_m]-_n$

$(C_1-C_3)alkyl[O(CH_2)_m]-_n$

wherein m represents the integer 2 or 3, n is an integer from 1 to 10, and one of the hydrogen atoms of the $-(CH_2)-$group may be substituted by a methyl group; $(C_2-C_{10})$alkanoyloxymethyl, phenyl, substituted phenyl, phenyl$(C_1-C_6)$alkyl, substituted phenyl$(C_1-C_6)$alkyl;

A represents hydrogen or $-N[(C_{10}C_{11})$aliphatic acyl]-beta-D-2-deoxy-2-amino-glucopyranosyl,
B represents hydrogen or N-acetyl-beta-D-2-deoxy-amino-glucopyranosyl
M represents hydrogen or alpha-D-mannopyranosyl;

and the addition salts thereof.

2. A compound of claim 1 wherein Y represents hydroxy or a group of formula $NR^1R^2$ wherein $R^1$ represents hydrogen or $(C_1-C_4)$alkyl, $R^2$ represents $(C_1-C_6)$alkyl, carboxy$(C_1-C_6)$alkyl, amino$(C_1-C_6)$alkyl, aminocarboxy$(C_1-C_6)$alkyl or a group $(C_1-C_6)$alkoxy and the salts thereof.

3. A compound of claim 1 which is 22-dechloroteicoplanin $A_2$ component 2.

4. A process for preparing a compound of claim 1, 2 or 3 which comprises reacting a teicoplanin derivative of formula II

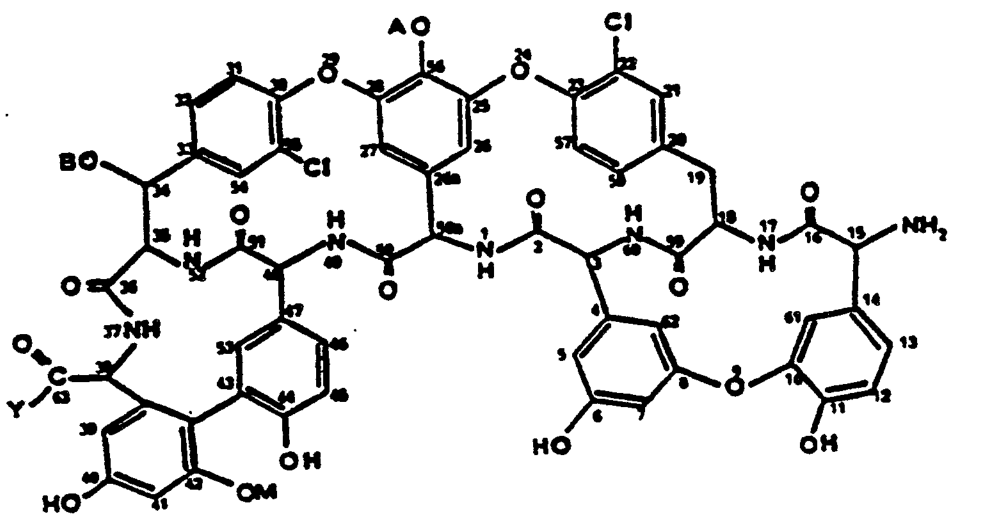

II

wherein Y, A, B and M are as defined in claim 1, with an alkali metal or earth alkali metal borohydride or cyanoborohydride in the presence of a Palladium-based hydrogenation catalyst in a polar organic solvent at a temperature between 10° C and 60° C.

5. A process according to claim 4 wherein the borohydride is sodium or potassium borohydride or sodium cyanoborohydride.

6. A process according to claim 4 wherein the Palladium based hydrogenation catalyst is Palladium chloride.

7. A process according to claim 4 wherein the reaction temperature is between 35° C and 40° C.

8. A compound of claim 1, 2 or 3 for use as a medicine.

9. A pharmaceutical composition comprising a compound of claim 1, 2 or 3 in admixture with a pharmaceutically acceptable carrier.

10. Use of a compound of claim 1, 2 or 3 for preparing a medicament for antibacterial use.

Claims for the following Contracting States: ES, GR

1. A process for preparing a teicoplanin derivative of formula

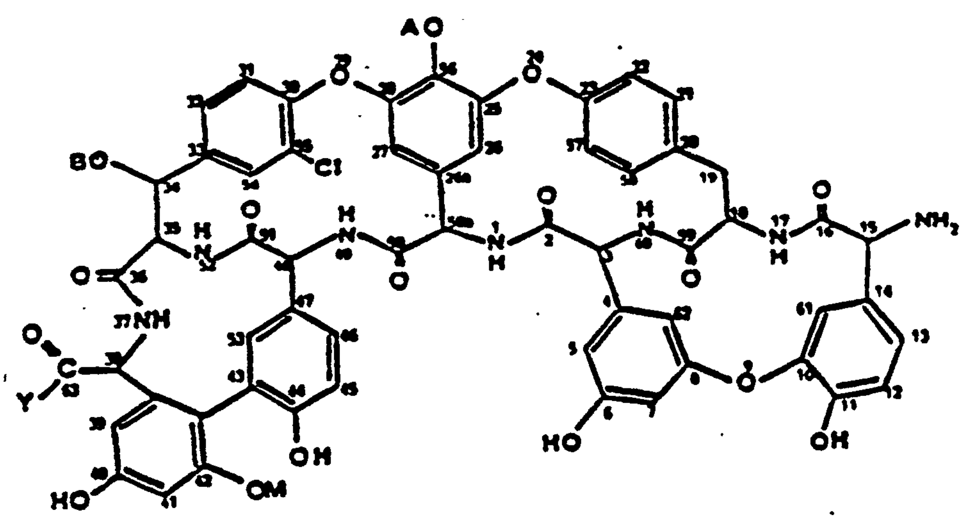

I

wherein:

Y represents hydroxy, a group $NR^1R^2$ wherein

$R^1$ represents hydrogen, $(C_1-C_6)$alkyl, hydroxy$(C_2-C_4)$alkyl, halogeno$(C_2-C_4)$alkyl, $(C_1-C_4)$alkoxy$(C_2-C_4)$-alkyl, amino$(C_2-C_4)$alkyl, $(C_1-C_4)$alkylamino$(C_2-C_4)$alkyl, di$(C_1-C_4)$alkylamino$(C_2-C_4)$alkyl

$R^2$ represents hydrogen, ($C_1$-$C_6$)alkyl, hydroxy($C_2$-$C_4$)alkyl, halogeno($C_2$-$C_4$)alkyl, ($C_1$-$C_4$)alkoxy($C_2$-$C_4$)-alkyl, a nitrogen containing 5-6 membered heterocyclic ring which may be unsaturated, partially saturated or wholly saturated and may contain 1 to 3 further heteroatoms selected from N, S and O wherein 1 to 3 of the ring carbons may optionally bear ($C_1$-$C_4$)alkyl substituents and one of the ring nitrogens may optionally bear a substituent $R^5$ selected from ($C_1$-$C_4$)alkyl, ($C_4$-$C_7$)cycloalkyl, phenyl optionally substituted with halogen or ($C_1$-$C_4$)alkyl, phenyl($C_1$-$C_4$)alkyl, pyridyl, ($C_1$-$C_4$)alkylpyridinio, and when the ring is wholly saturated two of the ring members may optionally be bridged by an alkylene chain of 1 to 3 carbon atoms wherein one of the methylene groups may optionally be replaced by -NH- or - N [($C_1$-$C_4$)alkyl];
a group -alk-W wherein "alk" represents a linear alkylene chain of 1 to 8 carbon atoms which is optionally substituted with a substituent selected from ($C_1$-$C_4$)alkyl, hydroxy($C_1$-$C_4$)alkyl, hydroxy, carboxy, aminocarbonyl, ($C_1$-$C_4$)alkylaminocarbonyl, di($C_1$-$C_4$)alkylaminocarbonyl, ($C_1$-$C_4$)alkoxycarbonyl, phenyl($C_1$-$C_4$)-alkoxycarbonyl, and W represents a carboxy, ($C_1$-$C_4$)alkoxycarbonyl, phenyl($C_1$-$C_4$)alkoxycarbonyl, aminocarbonyl, ($C_1$-$C_4$)aminocarbonyl, di($C_1$-$C_4$)aminocarbonyl, pentosaminocarbonyl, hexosaminocarbonyl, ureido, guanidino, a nitrogen containing 5-6 membered heterocyclic ring defined as above, a group of the formula

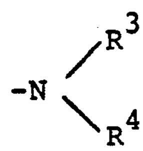

wherein $R^3$ and $R^4$ each independently represent hydrogen, ($C_1$-$C_6$)alkyl, hydroxy($C_2$-$C_4$)alkyl and halogeno($C_2$-$C_4$)alkyl, or $R^4$ represents phenylmethyloxycarbonyl and $R^3$ represents hydrogen; a group of the formula

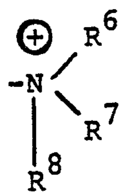

wherein $R^6$ , $R^7$ and $R^8$ each independently represent a ($C_1$-$C_4$)alkyl,

or $R^1$ and $R^2$ taken together with the adjacent nitrogen atom represent a saturated 5-7 membered heterocyclic ring which may optionally bear one to two ($C_1$-$C_4$)alkyl substituents on the ring carbons and may contain a further heterogroup selected from -O-, -S-, and -$NR^5$ - wherein $R^5$ is defined as above;

with the proviso that when W represents
a group

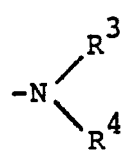 ,

a group

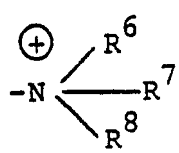 ,

ureido, guanidino or a nitrogen containing 5-6 membered heterocyclic ring as defined above directly connected with the "alk" moiety through a bond with a ring nitrogen atom, the linear alkylene "alk" moiety must be of at least two carbon atoms; or

Y represents -OR wherein R represents

$(C_1-C_{12})$alkyl, hydroxy$(C_1-C_{12})$alkyl, $(C_1-C_3)$alkoxy$(C_1-C_{12})$alkyl, halo$(C_1-C_{12})$alkyl; a group of formula

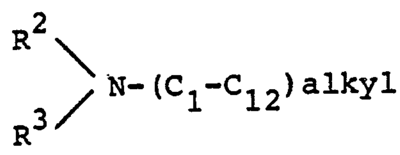

wherein $R^2$ and $R^3$ each independently represents hydrogen or $(C_1-C_4)$alkyl groups, or $R^2$ and $R^3$ taken together with the adjacent nitrogen atom represent a 5-7 membered aromatic, partially hydrogenated or saturated heterocycle ring which may optionally contain a further heteroatom selected from S, O and N; a group of formula

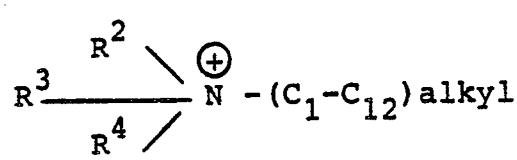

wherein $R^2$ and $R^3$ are as defined above and $R^4$ represents hydrogen or $(C_1-C_4)$alkyl; or R represents a group of formula

$H-[O(CH_2)_m]-_n$

$(C_1-C_3)alkyl[O(CH_2)_m]-_n$

wherein m represents the integer 2 or 3, n is an integer from 1 to 10, and one of the hydrogen atoms of the $-(CH_2)$-group may be substituted by a methyl group; $(C_2-C_{10})$alkanoyloxymethyl, phenyl, substituted phenyl, phenyl$(C_1-C_6)$alkyl, substituted phenyl$(C_1-C_6)$alkyl;

A represents hydrogen or $-N[(C_{10}-C_{11})$aliphatic acyl]-beta-D-2-deoxy-2-amino-glucopyranosyl,

B represents hydrogen or N-acetyl-beta-D-2-deoxy-amino-glucopyranosyl

M represents hydrogen or alpha-D-mannopyranosyl;

and the addition salts thereof, which comprises reacting a teicoplanin derivative of formula II

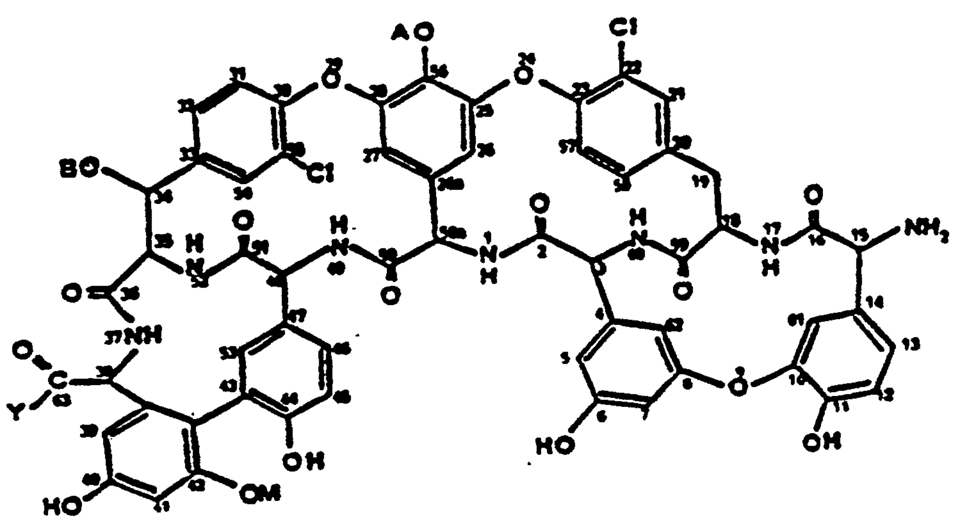

II

wherein Y, A, B and M are as defined above, with an alkali metal or earth alkali metal borohydride or cyanoborohydride in the presence of a Palladium-based hydrogenation catalyst in a polar organic solvent at a temperature between 10° C and 60° C.

2. A process according to claim 1 for preparing a compound wherein Y represents hydroxy or a group of formula $NR^1 R^2$ wherein $R^1$ represents hydrogen or $(C_1-C_4)$alkyl, $R^2$ represents $(C_1-C_6)$alkyl, carboxy$(C_1-C_6)$alkyl, amino$(C_1-C_6)$alkyl, aminocarboxy$(C_1-C_6)$alkyl or a group $(C_1-C_6)$alkoxy and the salts thereof.

3. A process according to claim 1 for preparing a compound which is 22-dechloroteicoplanin $A_2$ component 2.

4. A process according to claim 1, 2 or 3 wherein the borohydride is sodium or potassium borohydride or sodium cyanoborohydride.

5. A process according to claim 1, 2 or 3 wherein the Palladium based hydrogenation catalyst is Palladium chloride.

6. A process according to claim 1, 2 or 3 wherein the reaction temperature is between 35° C and 40° C.

7. Use of a compound of claim 1, 2 or 3 for preparing a medicament for antibacterial use.